# EUROPEAN PATENT SPECIFICATION

(11) **EP 1 915 058 B1**
(45) Date of publication and mention of the grant of the patent: **20.05.2015**
(21) Application number: 06795178.0
(22) Date of filing: 31.07.2006
(51) Int. Cl.: A23C 9/123, A61K 35/74, A23L 1/03

(54) **USE OF SPECIFIC LACTIC BACTERIA FOR THE PREPARATION OF IMMUNOMODULATING COMPOSITIONS**
VERWENDUNG SPEZIFISCHER LAKTOBAZILLEN ZUR HERSTELLUNG VON IMMUNOMODULATORISCHEN ZUSAMMENSETZUNGEN
UTILISATION DE BACTERIES LACTIQUES SPECIFIQUES POUR LA PREPARATION DE COMPOSITIONS IMMUNOMODULATRICES

(30) Priority: 02.08.2005 IT MI20051510
(43) Date of publication of application: 30.04.2008
(73) Proprietor: Proge Farm S.r.l., 28100 Novara (IT)
(72) Inventor: DONDI, Giancarla, 28100 Novara (IT); MALFA, Patrizia, 28100 Novara (IT)
(74) Representative: Trupiano, Federica
(86) International application number: PCT/IB2006/002080
(87) International publication number: WO 2007/015132

(56) References cited:
- EP-A2- 0 861 905
- US-A1- 2005 084 482
- PRIOULT GUENOLEE ET AL: "Stimulation of interleukin-10 production by acidic beta-lactoglobulin-derived peptides hydrolyzed with Lactobacillus paracasei NCC2461 peptidases" CLINICAL AND DIAGNOSTIC LABORATORY IMMUNOLOGY, vol. 11, no. 2, March 2004 (2004-03), pages 266-271, XP002409881 ISSN: 1071-412X
- FUJIWARA D ET AL: "A double-blind trial of Lactobacillus paracasei strain KW3110 administration for immunomodulation in patients with pollen allergy" ALLERGOLOGY INTERNATIONAL 2005 AUSTRALIA, vol. 54, no. 1, 1 March 2005 (2005-03-01), pages 143-149, XP002409882 ISSN: 1323-8930
- CASTELLAZZI ET AL: "F.18. ''In Vitro'' and ''in Vivo'' Immunomodulant Activity of PSMIX (Lactobacillus Paracasei I 1688 and Lactobacillus Salivarius I 1794)" CLINICAL IMMUNOLOGY, ACADEMIC PRESS,, US, vol. 119, 2006, page S57, XP005438340 ISSN: 1521-6616

## Description

### FIELD OF THE INVENTION

The present invention concerns the use of some particular lactobacteria for the preparation of compositions for the treatment of allergies and immunodeficiencies, more specifically the invention concerns use of the following two strains *Lactobacillus salivarius* I 1794 and *Lactobacillus paracasei* I 1688, alone or mixed with each other.

### TECHNICAL BACKGROUND

The influence of certain bacteria that produce lactic acid (LAB), present mainly in fermented food and probiotic preparations, on the immunity of animals and humans has been described. In particular, some LABs have proved to be capable of interacting with the immune system, modifying the type and degree of protection against pathogens and tumoural degenerations; an increase in the immune response at the level of the mucous membranes has also been demonstrated (Int. J. Immunopathol. Pharmacol., 2004; 17:127-134; Infection and Immunity, 2000; 68(2):752-59).

### DESCRIPTION OF THE INVENTION

It is also known that not all lactobacteria are probiotic and that only some have an effect on the immune system. *In vitro* immunology studies conducted with various strains of LAB have provided conflicting and in some respects antithetic results, demonstrating that not only is the existence of an activity of the LABs on the immune system non-predictable but also that some strains are able to inhibit the activity of other species of the same genus (J. Immunol., 2002, 168:171-178):

It has now been found that two specific strains of lactobacteria have a powerful immunomodulator effect, alone or combined, and are therefore useful for the treatment of pathologies associated with alterations of the immune system.

In particular it has surprisingly been found that two strains of lactobacteria *Lactobacillus salivarius* I 1794 and *Lactobacillus paracasei* I 1688 have a direct effect on the immune system, stimulating responses especially on the part of lymphocytes, T regulators and Natural Killer cells which produce a beneficial effect in subjects with a predisposition towards pathologies of the immune system, in particular but not only in allergic and/or immunodeficient subjects.

Thus, according to one of its embodiments, the invention concerns the use of at least one lactobacterium chosen from *Lactobacillus salivarius* I 1794, *Lactobacillus paracasei* I 1688 and a mixture thereof, for the preparation of a composition suitable for the treatment of allergies and immunodeficiencies.

The *Lactobacillus paracasei* I 1688 and *Lactobacillus salivarius* I 1794 were described for the first time in the European patent N° 0861905, granted on 24.11.2004, in the name of the same applicant for the treatment of disorders of the intestinal system.

"Mixture" according to the present invention indicates an association of the two strains of lactobacteria mentioned above, in any relative proportion.

According to a preferred embodiment, the mixture of the invention comprises the two lactobacteria specified above in a ratio of approximately 10:1, more preferably in the following respective proportions: 92% *Lactobacillus paracasei* I 1688 and 8% *Lactobacillus salivarius* I 1794.

The mixture comprising 92% *Lactobacillus paracasei* I 1688 and 8% *Lactobacillus salivarius* I 1794 is known per se for the treatment of disorders of the gastrointestinal tract such as dysmicrobism and is commonly and commercially called "PS MIX" (trademark application).

Other mixtures comprising different relative quantities of the two lactobacteria, if necessary combined with other lactobacteria or appropriate active agents, can also be used according to the invention.

The *Lactobacillus salivarius* I 1794, the *Lactobacillus paracasei* I 1688 and their mixtures as defined above, hereinafter also defined "active ingredients", are particularly useful as medicaments in the prevention and treatment of pathologies associated with allergy and immunodeficiencies.

It has in fact been demonstrated that said active ingredients are active in the stimulation of different immune cell types, more specifically in pathologies that involve a response of the lymphocytes, especially T-helper and T-cytotoxic lymphocytes with CD25, Natural Killer cells, B lymphocytes, dendritic cells and cytokines including, for example, TNFα, IFNγ, IL-10 and IL-12.

The details of the tests performed and the surprising results obtained with the active ingredients of the invention are given in the experimental section of the present description.

The *Lactobacillus salivarius* I 1794, the *Lactobacillus paracasei* I 1688 and their mixtures can therefore be used for example in the treatment and prevention of allergies and resulting pathologies.

The *Lactobacillus salivarius* I 1794, the *Lactobacillus paracasei* I 1688 and their mixtures can also be used in the treatment and prevention of immunodeficiencies of any origin and resulting pathologies.

Thus, the compositions of the invention may be used, advantageously in oral form, for the treatment of bacterial or viral infections, such as infections of the respiratory tract, infections of the gastrointestinal tract, infections of the mucous membranes, infections of the skin and all infections deriving from states of immunodeficiency.

In a condition of immunodeficiency, the defences of the organism against pathogens are reduced with consequent alteration of the Th1/Th2 balance as, for example, in physiological immunodeficiencies (newly-born babies, pregnancy), congenital immunodeficiencies (genetic diseases) and acquired immunodeficiencies (AIDS, autoimmune diseases).

The compositions of the invention can also be useful to support the natural immune defences of the organism, for example in particular states of stress such as psychophysical stress which, if excessively intense or protracted, can lead to a situation of immunodeficiency, clinically manifested by infectious forms of varying intensity.

To perform their action, the active ingredients of the invention, i.e. the *Lactobacillus salivarius* I 1794, the *Lactobacillus paracasei* I 1688 and their mixtures are preferably administered systemically, advantageously orally, in the form of compositions, possibly but not necessarily combined with one or more physiologically and/or pharmaceutically acceptable excipients or vehicles.

The term "compositions", according to the present invention, indicates any composition whether pharmaceutical, dietetic, alimentary or nutraceutic, advantageously oral, which comprises the lactobacteria described above or mixtures thereof.

Said compositions are prepared according to the known technique, taking account of the particular nature of the active ingredients of the invention consisting of living material, i.e. lactobacteria, which must therefore be treated so that it is able to survive processing, storage and administration.

According to a preferred embodiment, the compositions for use according to the invention are in the form of dosage units for administration once or several times a day, according to the type and severity of the pathology to be treated and the age and weight of the patient. In general said dosage units contain between 10³ and 10¹², advantageously between 10⁵ and 10¹⁰, for example between 10⁸ and 10¹⁰ CFU (colony forming units) of active ingredients per gram of composition.

For an adequate treatment, 1 to 3 dosage units, for example, are generally administered per day.

The active ingredients for use according to the invention are preferably administered orally and are in lyophilised form, included in suitable compositions possibly but not necessarily with excipients and conventional stabilisers, according to the methods well known to a person skilled in the art.

Thus for use according to the invention, the compositions containing the active ingredients are administered for an appropriate period of time, if necessary established by the specialist in charge, which varies in general between 1 week and 1 month or even more. Shorter or longer treatments can be taken into consideration, also in view of the non-toxicity of the active ingredients which permits the use thereof even in excess without dangerous side effects on the health of the subject treated.

The pharmaceutical, dietetic, alimentary and nutraceutic compositions according to the invention include any medicament, food, dietetic or nutraceutic product able to provide a vehicle for the lactobacteria described above in the organism. For purely illustrative purposes, these include, for example, drugs; dietetic products; products deriving from milk, such as yoghurt, cheese, cream; confectionery; fruit juices; etc.. Said compositions can comprise other beneficial substances for the organism such as, for example, vitamins, mineral salts, and/or other compatible active ingredients, for example prebiotic agents such as inulins, phospho-oligosaccharides (FOS), fibres, etc.

The following examples illustrate the invention without limiting it in any way.

### Example 1

Evaluation of direct activity on the cells of the systemic immune system of a mixture of *Lactobacillus salivarius* I 1794 and *Lactobacillus paracasei* I 1688

The proliferative response of the lymphocytes of the peripheral blood of some subjects as a response to the following doses of *Lactobacillus salivarius* I 1794 and *Lactobacillus paracasei* I 1688 and to two mixtures of the same was evaluated.

Before proceeding with the different evaluations described below, the bacteria underwent gamma irradiation for 24 hours with a source of cesium in order to block all spontaneous proliferation of the same.

The lymphocytes of 15 healthy subjects, isolated by Fycoll-Hypaque density gradient centrifugation (Lymphoprep,Nycomed Pharma AS,Oslo,Norway), resuspended in an RPMI 1640 medium (Gibco BRL, Life Technologues, Paisley, Scotland) with the addition of 2mM of L-glutamine, 50µg/m of gentamicin (Gibco) and 10% of fetal calf serum (FCS, Euroclone-Celbio,Milan,Italy) (RPMI-FCS) were placed in a culture (1X10⁵/200µl) in triplicate, in microplates with 96 U-bottom wells (non-stimulated cultures) or (stimulated cultures) with *Lactobacillus salivarius* (I 1794) (1X10⁵/200µl), *Lactobacillus paracasei* (I 1688) (1X10⁵/200µl), PS MIX (trademark application) (1X10⁵/200µl), or *Candida albicans* (2X10⁵/200µl) as a positive control, at 37°C in a humidified atmosphere, with 5% of CO₂. Eighteen hours after collection, 1µCi of [³H]TdR (Amershampharmacia Biotec, Milan, Italy) was added to each well.

The following ratios between bacteria/peripheral blood lymphocytes were used for the proliferation tests:
bacteria: lymphocytes
200:1
100:1
20:1
10:1
5:1
1:1
0.5:1
0.1:1
0.01:1
0.005:1
0.001:1
compared with the proliferative response of Candida albicans at two ratios (0.2:1 and 2:1) in a six-day cell culture, at 37°C in an atmosphere with 5% of CO₂.

The results were expressed as Stimulation Index (SI: cpm of stimulated cultures /cpm of non-stimulated cultures, where cpm means "counts per minute") and indicate measurement of the recognition by the lymphocytes of the bacterial antigens and the ability to respond, by proliferating, to the antigen recognition.

### Results

An excellent proliferative response to the bacteria/cell co-culture was observed denoting recognition by the lymphocytes of the antigen determinants of the lactobacteria and lymphocyte activation following said recognition.

### Example 2

### Phenotype of the lymphocytic subpopulations

The phenotype of the lymphocytic subpopulations was evaluated at time 0, before culture of the bacteria, and after 6 days' culture to evaluate which phenotype was induced.

Monoclonal antibodies anti-CD3, anti-CD4, anti-CD8, anti-CD25, anti-HLA were used to evaluate the T lymphocytic populations and subpopulations, anti-CD16 and anti-CD56 to evaluate the Natural Killers, anti-CD20, anti-CD38 and anti-CD79 for the lymphocytic B populations and the plasmacells. The reading was performed with a tricolour method.

### Results

A substantial increase in the Natural Killers was observed, which, as is known, provide a better response to the cells infected by viruses and the neoplastically transformed cells.

An increase in the T-lymphocyte helpers and cytotoxic T-lymphocytes with CD25 membrane, which represent the subpopulations most directly affected by a modulation of the immune response, was also observed.

### Example 3

The production of some cytokines was ascertained in the buffy coat of the cell cultures via an ELISA method developed for each cytokine (TNF-alfa, IFN-gamma, IL-10, IL-12, IL-4).

### Results

The production of IL-4 in the supernatant in response to activation of the lymphocytes by the lactobacteria was below the levels measurable with the ELISA test, indicating non-activation of the T-helpers 2 (Th2) responsible for induction of the allergenic phenomena.

High TNF-alfa and IFN-gamma values were found, on the other hand, indicating activation of the T-helpers 1 (Th1).

Considerable quantities of IL-10 and IL-12 were also found; as is known, the first induces differentiation of the B lymphocytes, inhibits activation of the macrophages and protects against the risk of inflammatory bowel disease (IBD), and the second activates the Natural Killers inducing the production of IFN-gamma, a crucial agent in the first phases of an infection.

### Example 4

An oral composition is prepared, in powder form, comprising the following components for each dosage unit:
PS MIX (trademark application) (lyophilised) 1 x 10⁸ - 1 x 10¹⁰
Sucrose; Malt dextrins; Aroma; Silica; Vitamin B2; Vitamin B1.

## Claims

1. A mixture of *Lactobacillus salivarius* I 1794 and *Lactobacillus paracasei* I 1688 for use in the prevention and treatment of allergies and in the prevention and treatment of immunodeficiencies.

2. The mixture for use as claimed in claim 1, **characterised in that** said mixture is used in any proportion.

3. The mixture for use as claimed in claim 2, **characterised in that** said mixture comprises 8% of *Lactobacillus salivarius* I 1794 and 92% of *Lactobacillus paracasei* I 1688.

4. The mixture for use as claimed in claims 1 to 3, wherein said mixture in combined with other lactobacteria or active agents.

5. The mixture for use as claimed in one of the claims from 1 to 4, **characterised in that** said mixture is administered in the form of oral compositions if necessary with one or more physiologically and/or pharmaceutically acceptable excipients and/or vehicles.

6. The mixture for use as claimed in one of the claims from 1 to 5, **characterised in that** said mixture is administered in the amount of at least 1 x 10⁸ - 1 x 10¹⁰ CFU per day.

7. The mixture for use as claimed in one of the claims from 1 to 6, **characterised in that** said mixture is administered after reconstitution of the lyophilised form.

8. The mixture for use as claimed in one of the claims from 1 to 7, **characterised in that** said mixture is administered in the form of dosage units.

9. Pharmaceutical, dietetic, alimentary or nutraceutical composition comprising a mixture as defined in anyone of claims 1 to 4 for use in the prevention and treatment of allergies and in the prevention and treatment of immunodeficiencies.

10. The composition for use according to claim 9, which is for oral administration.

11. The composition for use according to claims 9 or 10, in the form of dosage units comprising between 10³ and 10¹² CFU of said mixture.

12. The composition for use according to claim 11, in the form of dosage units comprising between 10⁸ and 10¹⁰ CFU of said mixture.

## Patentansprüche

1. Eine Mischung von *Lactobacillus salivarius* I 1794 und *Lactobacillus paracasei* I 1688 zur Verwendung in der Prävention und Behandlung von Allergien und in der Prävention und Behandlung von Immundefekten.

2. Mischung zur Verwendung gemäß Anspruch 1, **dadurch gekennzeichnet, dass** die Mischung in einem beliebigen Verhältnis verwendet wird.

3. Mischung zur Verwendung gemäß Anspruch 2, **dadurch gekennzeichnet, dass** die Mischung 8 % *Lactobacillus salivarius* I 1794 und 92 % *Lactobacillus paracasei* I 1688 umfasst.

4. Mischung zur Verwendung gemäß einem der Ansprüche 1 bis 3, wobei die Mischung mit anderen Milchsäurebakterien oder aktiven Wirkstoffen kombiniert ist.

5. Mischung zur Verwendung gemäß einem der Ansprüche 1 bis 4, **dadurch gekennzeichnet, dass** die Mischung in Form von oralen Zusammensetzungen verabreicht wird, sofern nötig mit einem oder mehreren physiologisch und/oder pharmazeutisch annehmbaren Hilfsstoffen und/oder Trägerstoffen.

6. Mischung zur Verwendung gemäß einem der Ansprüche 1 bis 5, **dadurch gekennzeichnet, dass** die Mischung in der Menge von mindestens 1 x 10⁸ bis 1 x 10¹⁰ CFU pro Tag verabreicht wird.

7. Mischung zur Verwendung gemäß einem der Ansprüche 1 bis 6, **dadurch gekennzeichnet, dass** die Mischung nach Rekonstitution der lyophilisierten Form verabreicht wird.

8. Mischung zur Verwendung gemäß einem der Ansprüche 1 bis 7, **dadurch gekennzeichnet, dass** die Mischung in der Form von Dosierungseinheiten verabreicht wird.

9. Pharmazeutische, diätische, zur Nahrung dienende oder nutrazeutische Zusammensetzung, umfassend eine Mischung gemäß einem der Ansprüche 1 bis 4, zur Verwendung in der Prävention und Behandlung von Allergien und in der Prävention und Behandlung von Immundefekten.

10. Zusammensetzung zur Verwendung gemäß Anspruch 9, welche für eine orale Verabreichung ist.

11. Zusammensetzung zur Verwendung gemäß einem der Ansprüche 9 oder 10, in der Form von Dosiereinheiten, umfassend zwischen 10³ und 10¹² CFU der Mischung.

12. Zusammensetzung zur Verwendung gemäß Anspruch 11, in der Form von Dosiereinheiten, umfassend zwischen 10⁸ und 10¹⁰ CFU der Mischung.

## Revendications

1. Mélange de *Lactobacillus salivarius* I 1794 et de *Lactobacillus paracasei* I 1688 pour son utilisation dans la prévention et le traitement d'allergies et dans la prévention et le traitement de déficiences immunitaires.

2. Mélange pour son utilisation selon la revendication 1, **caractérisé en ce que** ledit mélange est utilisé en toute proportion.

3. Mélange pour son utilisation selon la revendication 2, **caractérisé en ce que** ledit mélange comprend 8 % de *Lactobacillus salivarius* I 1794 et 92 % de *Lactobacillus paracasei* I 1688.

4. Mélange pour son utilisation selon les revendications 1 à 3, dans lequel ledit mélange est combiné avec d'autres lactobactéries ou agents actifs.

5. Mélange pour son utilisation selon l'une des revendications 1 à 4, **caractérisé en ce que** ledit mélange est administré sous la forme de compositions orales au besoin avec un ou plusieurs excipients et/ou véhicules physiologiquement et/ou pharmaceutiquement acceptables.

6. Mélange pour son utilisation selon l'une des revendications 1 à 5, **caractérisé en ce que** ledit mélange est administré en la quantité d'au moins 1 x 10⁸ à 1 x 10¹⁰ UFC par jour.

7. Mélange pour son utilisation selon l'une des revendications 1 à 6, **caractérisé en ce que** ledit mélange est administré après la reconstitution de la forme lyophilisée.

8. Mélange pour son utilisation selon l'une des revendications 1 à 7, **caractérisé en ce que** ledit mélange est administré sous la forme d'unités galéniques.

9. Composition pharmaceutique, diététique, alimentaire ou nutraceutique comprenant un mélange selon l'une quelconque des revendications 1 à 4, pour son utilisation dans le traitement d'allergies et dans la prévention et le traitement de déficiences immunitaires.

10. Composition pour son utilisation selon la revendication 9, qui est destinée à une administration orale.

11. Composition pour son utilisation selon la revendication 9 ou la revendication 10, sous la forme d'unités galéniques comprenant de 10³ à 10¹² UFC dudit mélange.

12. Composition pour son utilisation selon la revendication 11, sous la forme d'unités galéniques comprenant de 10⁸ à 10¹⁰ UFC dudit mélange.
